# EUROPÄISCHE PATENTSCHRIFT

(11) **EP 2 829 296 B1**
(45) Veröffentlichungstag und Bekanntmachung des Hinweises auf die Patenterteilung: **15.08.2018**
(21) Anmeldenummer: 14173845.0
(22) Anmeldetag: 25.06.2014
(51) Int. Cl.: A61M 5/32

(54) **Medizinische Injektionsvorrichtung**
Medical injection device
Dispositif médical d'injection

(30) Priorität: 24.07.2013 DE 102013214429
(43) Veröffentlichungstag der Anmeldung: 28.01.2015
(73) Patentinhaber: RAUMEDIC AG, 95213 Münchberg (DE)
(72) Erfinder: Jakob, Thomas, 95111 Rehau (DE); Maag, Sebastian, 95448 Bayreuth (DE); Festel, Tobias, 95234 Sparneck (DE); Braun, Thomas, 95032 Hof (DE); Skaper, Frank, 95191 Leupoldsgrün (DE)
(74) Vertreter: Rau, Schneck & Hübner Patentanwälte Rechtsanwälte PartGmbB

(56) Entgegenhaltungen:
- EP-A1- 2 578 255
- EP-A1- 2 578 256
- EP-A2- 0 272 035
- WO-A1-2014/048298
- US-A- 4 923 447
- US-A1- 2009 259 193

## Beschreibung

Die Erfindung betrifft eine medizinische Injektionsvorrichtung mit einer Injektionseinrichtung und einer Stechschutzeinrichtung.

Medizinische Injektionsvorrichtungen mit Stechschutz sind bekannt aus der EP 460 914 B1, der EP 707 860 B1, der US 4,838,871, der EP 692 271 B1, der US 4,944,397, der US 4,982,842, der US 5,232,455, der US 5,139,489, der US 5,154,285, der US 5,277,311, der US 5,232,454, der US 5,312,367, der US 5,342,322, der US 5,423,765, der US 5,643,219, der WO 1991/009639 A2, der EP 862 920 B1, der EP 885 621 B1, der EP 1 525 016 B1, der EP 1 568 321 A1, der EP 1 587 419 B1, der EP 1 592 346 B1, der US 5,584,816, der US 5,632,732, der CH 685 979 A5 und der DE 695 02 357 T2.

Die US 2009/0259193 A1 beschreibt eine Einweg-Spritze.

Die EP 2 578 256 A1, EP 2 578 255 A1 und US 4,923,447 A beschreiben eine Nadelschutzeinrichtung.

Die EP 0 272 035 A2 beschreibt eine Injektionseinrichtung.

Die in Bezug auf das Prioritätsdatum dieser Anmeldung nachveröffentlichte WO 2014/048298 A1 beschreibt eine Nadelschutzeinrichtung für eine Spritze vom Typ Gleithülse.

Es ist eine Aufgabe der vorliegenden Erfindung, eine Injektionsvorrichtung der eingangs genannten Art derart weiterzubilden, dass eine intuitiv handhabbare und betriebssichere Stechschutzeinrichtung resultiert. Diese Aufgabe ist erfindungsgemäß gelöst durch eine medizinische Injektionsvorrichtung mit einer Stechschutzeinrichtung und einer Sicherungseinrichtung mit den im Anspruch 1 angegebenen Merkmalen.

Die Sicherungseinrichtung führt zu einer sicheren Beibehaltung der Schutzstellung durch die Schutzkomponente, sobald die Schutzstellung der Schutzkomponente erreicht ist. Die Sicherungseinrichtung kann als rastende Sicherungseinrichtung, zum Beispiel als axial und/oder radial rastende Sicherungseinrichtung ausgestaltet sein. Alternativ oder zusätzlich kann die Sicherungseinrichtung eine sichernde Festlegung der Schutzkomponente in der Schutzstellung durch Formschluss erreichen, beispielsweise durch Gestaltung nach Art einer Bajonett-Verbindung. Die Stechschutzeinrichtung kann aktiv von der Injektionsstellung in die Schutzstellung verlagerbar sein und kann so gestaltet sein, dass sie vom Anwender zu verlagern ist. Die komplette Stechschutzeinrichtung und auch die Sicherungseinrichtung können mit Kunststoffkomponenten realisiert sein. Die medizinische Injektionsvorrichtung hat einen Formschluss-Adapter zur Formschlussverbindung der Stechschutzeinrichtung mit der Injektionseinrichtung. Ein derartiger Formschluss-Adapter kann so ausgeführt sein, dass die Stechschutzeinrichtung an handelsübliche Gestaltungen von Injektionseinrichtungen angepasst werden kann. Eine Formschluss- und/oder Rastverbindung des Formschluss-Adapters kann sowohl mit der Injektionseinrichtung als auch mit der Stechschutzeinrichtung erfolgen. Ein Verdrehschutz des Formschluss-Adapters kann sowohl mit der Injektionseinrichtung als auch mit der Stechschutzeinrichtung gewährleistet sein. Der Formschluss-Adapter kann als axial aufsteckbare Adapterhülse oder als radial aufrastbarer, C-förmiger Adapter ausgeführt sein. Der Formschluss-Adapter kann gleichzeitig eine Verbindungs-Hülse bei einer Teleskop-Ausführung der Stechschutzeinrichtung darstellen. Bei dieser Verbindungs-Hülse kann es sich um eine der Teleskophülsen handeln. Die Stechschutzeinrichtung hat mindestens zwei Teleskophülsen, wobei eine Verbindungs-Teleskophülse mit der Injektionseinrichtung verbunden ist und wobei eine Schutz-Teleskophülse die Schutzkomponente darstellt. Eine derartige Ausführung als Teleskop-Stechschutzeinrichtung lässt sich kompakt realisieren. In der Injektionsstellung können die Teleskophülsen übereinander geschoben angeordnet sein. In der Schutzstellung können die Teleskophülsen teleskopiert angeordnet sein. Die Injektionskanüle kann von der Schutz-Teleskophülse vollständig und nach allen Seiten umschlossen sein. Zwischen den Teleskophülsen kann eine Formschluss- und/oder Rastanordnung eine Verbindung herstellen. Mindestens eine der Teleskophülsen, die in einer anderen der Teleskophülsen angeordnet ist, kann eine konisch verlaufende Außenwandung aufweisen. Dies kann eine gleichmäßige Kraftentfaltung bei einer Verlagerung der Formschluss- und/oder Rastanordnung zwischen der Injektionsstellung und der Schutzstellung gewährleisten. Dies kann eine intuitive Handhabung der Stechschutzeinrichtung bei der Überführung von der Injektionsstellung in die Schutzstellung verbessern. Zwischen einer Rastposition, die die Injektionsstellung zwischen den Teleskopkomponenten vorgibt, und einer Raststellung, die die Schutzstellung der Teleskopkomponenten vorgibt, kann mindestens eine Rast-Zwischenstellung durch entsprechende Rast-Zwischenstufen vorgegeben sein. Die Rast-Zwischenstufen können insbesondere mit Umfangsnuten in Außenwandungen der jeweils innenliegenden Teleskophülsen ausgeführt sein. Die Rastanordnung kann als radial und/oder als axial wirkende Rastverbindung gestaltet sein. Die Richtungsangaben "radial" und "axial" beziehen sich dabei auf die Bewegungsrichtung von Rastkomponenten bei der Verlagerung zwischen einer Raststellung und einer Freigabestellung in Bezug auf eine Mittel-Längsachse der Stechschutzeinrichtung.

Eine Mehrkomponenten-Spritzgussteil-Ausführung erweitert die Möglichkeiten zur Gestaltung der Bauteile der Stechschutzeinrichtung. Das Mehrkomponenten-Spritzgussteil kann als Zweikomponenten-Spritzgussteil oder auch als Spritzgussteil mit mehr als zwei Komponenten, beispielsweise drei Komponenten, vier Komponenten, fünf Komponenten oder noch mehr Komponenten, ausgeführt sein. Es können weichere Kunststoffe mit härteren Kunststoffen kombiniert werden. Weichere Kunststoffe können beispielsweise für einen Griffabschnitt der Stechschutzeinrichtung oder für durch Anlage an Gegenkomponenten wirkende Anformbauteile zum Spielausgleich und/oder zur Schaffung bzw. Erhöhung einer Reibschlusswirkung zwischen dem jeweiligen Anformbauteil und der jeweiligen Gegenkomponente genutzt werden.

Eine Weichkomponente des Mehrkomponenten-Spritzgussteils kann beispielsweise aus einem oder mehrerer thermoplastischer Elastomere (TPE), aus Polyurethan oder aus Silikon gefertigt sein. Eine Hartkomponente des Mehrkomponenten-Spritzgussteils kann aus Polypropylen, aus Polyethylen, aus ABS (Acrylnitril-Butadien-Styrol), aus einem Thermoplasten auf Basis von Methylmethacrylat, Acrylnitril, Butadien und Styrol (MABS), aus Polyoxymethylen (POM), aus Polybutylenterephthalat (PBT) oder auch als Blendsystem, also als Mischung, auf Basis von Polyolefinen sowie Polyamid gefertigt sein.

Eine Schutz-Rastanordnung nach Anspruch 2 führt zu einer besonders sicheren Festlegung der Schutzkomponente in der Schutzstellung. Die Schutz-Rastanordnung kann so ausgeführt sein, dass die Schutzkomponente, sobald sie einmal in die Schutzstellung überführt ist, zerstörungsfrei vom Anwender aus der Schutzstellung nicht mehr verlagert werden kann.

Eine Injektions-Verbindungsanordnung nach Anspruch 3 verhindert, dass die Stechschutzeinrichtung ungewollt von der Injektionsstellung in die Schutzstellung überführt wird. Die Injektions-Verbindungsanordnung kann rastend, zum Beispiel axial und/oder radial rastend, und/oder durch Formschluss, zum Beispiel als Bajonett-Verbindung, ausgeführt sein, wie vorstehend im Zusammenhang mit der Schutz-Rastanordnung bereits erläutert.

Eine Injektions-Rastanordnung nach Anspruch 4 lässt sich mit geringem Herstellungsaufwand realisieren. Die Injektions-Rastanordnung kann überwindbar ausgeführt sein, was beispielsweise durch Ausklinken von Rastkomponenten aufgrund definierten Drucks durch den Anwender auf die Stechschutzeinrichtung erreichbar ist.

Mindestens eine weitere Teleskophülse nach Anspruch 5 ermöglicht einen größeren Hub der Teleskop-Stechschutzeinrichtung zwischen der Schutzstellung und der Injektionsstellung und damit bei kompakter axialer Baulänge dennoch das Überdecken längerer Injektionskanülen. Zwischen der Verbindungs-Teleskophülse und der Schutz-Teleskophülse kann genau eine weitere Teleskophülse angeordnet sein. Auch mehrere derartige weitere Teleskophülsen, zum Beispiel zwei oder drei weitere Teleskophülsen, können bei einer derartigen Teleskop-Stechschutzeinrichtung vorgesehen sein. Eine Schutz-Rastanordnung nach Anspruch 6 kann Rastzähne mit einer Vorzugsrichtung aufweisen, um hierdurch eine Einweg-Umstellung der Schutzkomponente der Stechschutzeinrichtung in die Schutzstellung zu gewährleisten und/oder um eine definierte Endposition der Schutzkomponente in der Schutzstellung zu gewährleisten. Die Schutz-Rastanordnung kann mehrere in Umfangsrichtung um die Teleskophülsen angeordnete Rast-Zahnreihen aufweisen, die jeweils mit einem Gegenrastkörper zusammenwirken können. Die Rastzähne können einstückig an die jeweilige Teleskophülse angeformt sein.

Mindestens eine Nut/Feder-Einrichtung nach Anspruch 7 gewährleistet eine definierte und sichere Führung zwischen den Teleskophülsen bei deren Relativverlagerung zwischen der Injektionsstellung und der Schutzstellung.

Die Ausführungen nach Anspruch 8 geben vorteilhafte Varianten beim Einsatz von Mehrkomponenten-Spritzguss-Bauteilen an.

Eine Mehrkomponenten-Spritzguss-Gestaltung des Formschluss-Elements und des Formschluss-Element-Tragkörpers ermöglicht es, das mindestens eine Formschluss-Element, das als Axialrippe ausgeführt sein kann, aus einem im Vergleich zum Formschluss-Element-Tragkörper weicheren Kunststoffmaterial auszuführen, so dass ein Reibschluss der Formschluss-Elemente am inneren Mündungs- und Verbindungsabschnitt verbessert ist. Dies verbessert die Verdrehsicherheit des Formschluss-Adapters relativ zum Mündungs- und Verbindungsabschnitt.

Ausführungsbeispiele der Erfindung werden nachfolgend anhand der Zeichnung näher erläutert. In dieser zeigen:
- Fig. 1: eine medizinische Injektionsvorrichtung mit einer Injektionseinrichtung und einer Teleskop-Stechschutzeinrichtung in montiertem und auslieferungsfertigem Zustand;
- Fig. 2: die Injektionsvorrichtung nach Fig. 1 in benutzungsfertigem Zustand, bei dem eine ursprüngliche Schutzkappe von einer Injektionskanüle abgenommen ist;
- Fig. 3: die Injektionsvorrichtung nach Fig. 2 während einer Verlagerung der Teleskop-Stechschutzeinrichtung zwischen einer Injektionsstellung nach den Fig. 1 und 2, in der die Injektionskanüle zum Injizieren eines Mediums freilegbar (Fig. 1) oder freigelegt (Fig. 2) ist, und einer Schutzstellung, in der eine Kanülenspitze der Injetionskanüle in einer Schutzkomponente der Teleskop-Stechschutzeinrichtung versenkt angeordnet ist;
- Fig. 4: die Injektionsvorrichtung mit der TeleskopStechschutzeinrichtung in der Schutzstellung;
- Fig. 5: einen Teil-Längsschnitt durch die Injektionsvorrichtung mit aufgesetzter ursprünglicher Schutzkappe, wobei die Teleskop-Stechschutzeinrichtung in einer Stellung zwischen der Injektionsstellung und der Schutzstellung dargestellt ist;
- Fig. 6: eine Ausschnittsvergrößerung des Details VI in Fig. 5;
- Fig. 7: eine vergrößerte Ansicht eines Ausschnitts der Injektionsvorrichtung in der Schutzstellung;
- Fig. 8 bis 11: Momentanpositionen bei der Montage der Injektionsvorrichtung;
- Fig. 12: eine Explosionsdarstellung einer weiteren Ausführung einer medizinischen Injektionsvorrichtung mit einer Teleskop-Stechschutzeinrichtung;
- Fig. 13: die Injektionsvorrichtung nach Fig. 12 mit der Stechschutzeinrichtung in der Injektionsstellung;
- Fig. 14: die Injektionsvorrichtung nach Fig. 12 mit der Stechschutzeinrichtung in der Schutzstellung;
- Fig. 15: einen Formschluss-Adapter zur Formschlussverbindung der Stechschutzeinrichtung nach den Fig. 12 bis 14 mit der Injektionseinrichtung, ausgeführt als radial aufrastbarer, C-förmiger Adapter;
- Fig. 16: eine Teleskophülse der Stechschutzeinrichtung nach den Fig. 12 bis 14, die zwischen den Formschluss-Adapter nach Fig. 15 und einer Schutz-Teleskophülse der Stechschutzeinrichtung angeordnet ist;
- Fig. 17: die Schutz-Teleskophülse der Stechschutzeinrichtung nach den Fig. 12 bis 14;
- Fig. 18: eine Explosionsdarstellung einer weiteren Ausführung einer Teleskop-Stechschutzeinrichtung für eine medizinische Injektionsvorrichtung;
- Fig. 19: eine Injektionsvorrichtung mit der Stechschutzeinrichtung nach Fig. 18 in der Injektionsstellung;
- Fig. 20: die Injektionsvorrichtung nach Fig. 18 mit der Stechschutzeinrichtung in der Schutzstellung;
- Fig. 21: einen Formschluss-Adapter zur Formschlussverbindung der Stechschutzeinrichtung nach den Fig. 18 bis 20 mit der Injektionseinrichtung;
- Fig. 22: eine Verbindungs-Teleskophülse der Stechschutzeinrichtung nach den Fig. 18 bis 20, die zwischen dem Formschluss-Adapter nach Fig. 21 und einer mittleren Teleskophülse der Stechschutzeinrichtung angeordnet ist;
- Fig. 23: eine mittlere Teleskophülse der Stechschutzeinrichtung nach den Fig. 18 bis 20, die zwischen der Verbindungs-Teleskophülse nach Fig. 22 und einer Schutz-Teleskophülse der Stechschutzeinrichtung angeordnet ist;
- Fig. 24: die Schutz-Teleskophülse der Stechschutzeinrichtung nach den Fig. 18 bis 20;
- Fig. 25: einen ringförmigen Deckel für die Schutz-Teleskophülse nach Fig. 24;
- Fig. 26: einen axialen Längsschnitt durch die Stechschutzeinrichtung nach den Fig. 18 bis 20, dargestellt in der Schutzstellung;
- Fig. 27 bis 35: zu den Fig. 18 bis 26 ähnliche Darstellungen von Bauteilen einer weiteren Ausführung einer Stechschutzeinrichtung für eine Injektionseinrichtung;
- Fig. 36 bis 42: zu den Fig. 18 bis 24 ähnliche Darstellungen von Bauteilen einer weiteren Ausführung einer Stechschutzeinrichtung für eine Injektionseinrichtung; und
- Fig. 43: einen axialen Längsschnitt durch die Stechschutzeinrichtung nach den Fig. 36 bis 38, dargestellt in der Schutzstellung.

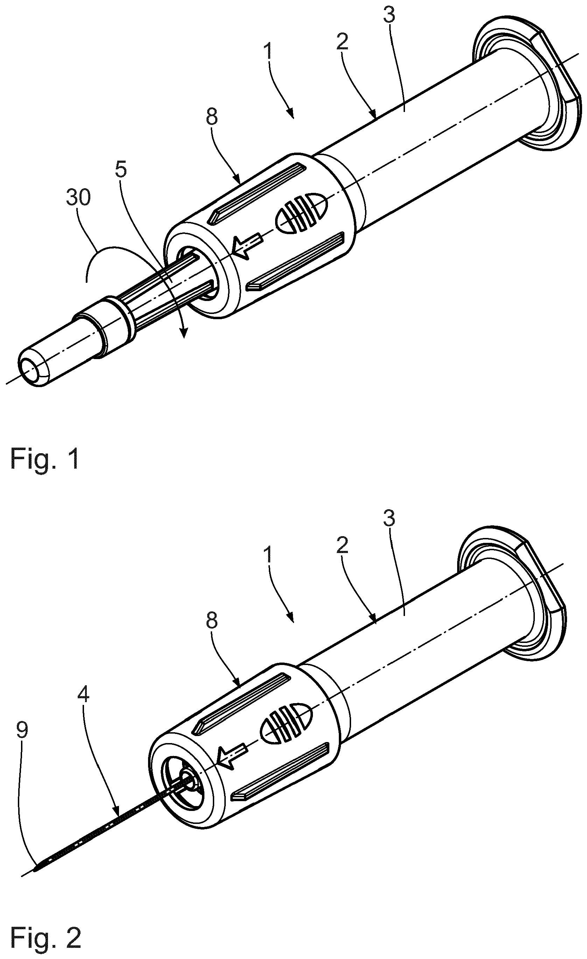

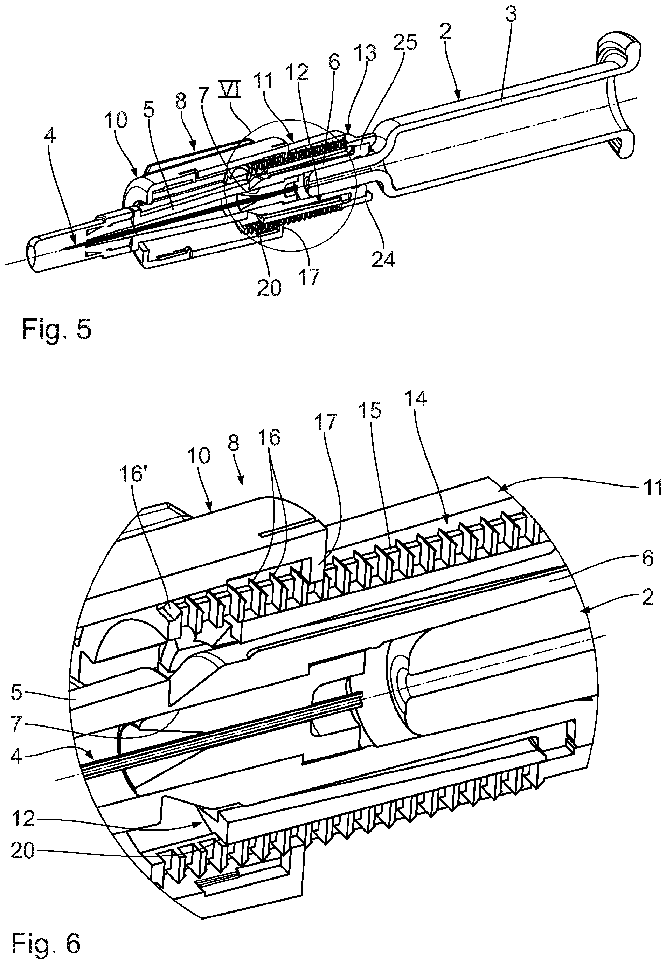

Fig. 1 bis 11 zeigen eine Ausführung einer medizinischen Injektionsvorrichtung 1. Die Injektionsvorrichtung 1 hat eine Injektionseinrichtung 2. Zu dieser gehört ein Behälter 3 für das zu injizierende Medium. Der Behälter 3 kann als Spritzenbehälter 3 zur Aufnahme eines Spritzenkolbens ausgeführt sein, der in der Zeichnung nicht dargestellt ist. Zur Injektionseinrichtung 2 gehört weiterhin eine Injektionskanüle 4, die in der Fig. 2 sichtbar ist und die in der Fig. 1 von einer ursprünglichen Schutzkappe 5 abgedeckt ist. Die ursprüngliche Schutzkappe 5 ist im ursprünglich ausgelieferten Zustand der Injektionsvorrichtung 1 nach Fig. 1 auf die Injektionskanüle 4 aufgesetzt und mit einem kanülenseitigen Ende des Behälters 3 axial verrastet. Zum Injizieren des Mediums kommuniziert die Injektionskanüle 4 mit dem Behälter 3 über einen kanülenseitigen Mündungs- und Verbindungsabschnitt 6 des Behälters 3, der in der Schnittdarstellung nach Fig. 5 sichtbar ist. Der Mündungs- und Verbindungsabschnitt 6 ist auf ein konisch zulaufendes Mündungsende eines Glaskörpers des Behälters 3 aufgeschoben und kann mit diesem zusätzlich formschlüssig und insbesondere rastend verbunden sein. Die Injektionskanüle 4, bei der es sich um eine metallische Kanüle handelt, ist mit dem Mündungsabschnitt 6 über eine Steck- bzw. Konusverbindung 7 verbunden. Bei einer nicht dargestellten Variante der Injektionsvorrichtung 1 ist die Konusverbindung 7 als Luerlock-Verbindung gestaltet. Eine Innenwand des Mündungsabschnitts 6 kann im Bereich der Steck- bzw. Konusverbindung 7 mit einer Außenwand eines Kanülenansatzes verrastet oder in sonstiger Weise formschlüssig verbunden sein.

Abgesehen von der Injektionskanüle 4 sind sämtliche Komponenten der Injektionsvorrichtung 1 aus Kunststoff gefertigt. Grundsätzlich kann auch die Injektionskanüle 4 aus Kunststoff gefertigt sein.

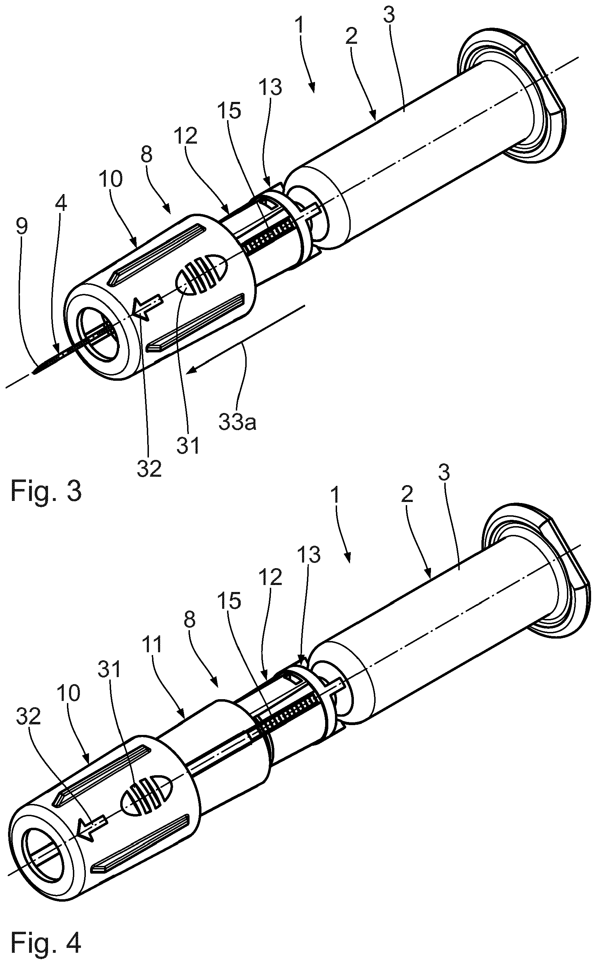

Die Injektionsvorrichtung 1 hat weiterhin eine Stechschutzeinrichtung 8. Diese ist verlagerbar zwischen einer in der Fig. 2 dargestellten Injektionsstellung, in der die Injektionskanüle 4 zum Beispiel zum subkutanen oder intravenösen Injizieren des Mediums freilegbar ist, und einer in der Fig. 4 dargestellten Schutzstellung, in der eine Kanülenspitze 9 der Injektionskanüle 4 in einer Schutzkomponente 10 der Stechschutzeinrichtung 8 versenkt angeordnet ist.

Die Stechschutzeinrichtung 8 umgibt den Mündungsabschnitt 6 in Form einer Hülse und hat mindestens zwei Teleskophülsen. Bei der Ausführung nach den Fig. 1 bis 11 hat die Stechschutzeinrichtung 8 insgesamt drei Teleskophülsen 10, 11 und 12, wobei eine dieser drei Teleskophülsen, die Schutz-Teleskophülse 10, die Schutzkomponente der Stechschutzeinrichtung 8 darstellt. Die Schutz-Teleskophülse 10 ist gleichzeitig die äußerste der drei Teleskophülsen 10 bis 12 der Stechschutzeinrichtung 8. Eine innerste der drei Teleskophülsen, die Verbindungs-Teleskophülse 12, ist über einen Formschluss-Adapter 13 mit der Injektionseinrichtung 2 verbunden. Zwischen der innersten Teleskophülse 12 und der äußersten Teleskophülse 10 der Stechschutzeinrichtung 8 liegt die Teleskophülse 11 als weitere Teleskophülse der Stechschutzeinrichtung 8.

In der Injektionsstellung sind die Teleskophülsen 10 bis 12 vollständig übereinander geschoben. In der Injektionsstellung überdeckt die Stechschutzeinrichtung 8 die Konusverbindung 7 axial, so dass diese von außen her nicht zugänglich ist. In der Schutzstellung sind die Teleskophülsen 10 bis 12 relativ zueinander teleskopiert.

Die Fig. 5 und 6 zeigen Details der Injektionsvorrichtung 1 und insbesondere Details der Stechschutzeinrichtung 8.

Zum sichernden Festlegen der Schutzkomponente, also der äußersten Schutz-Teleskophülse 10, in der Schutzstellung dient eine Sicherungseinrichtung in Form einer Schutz-Rastanordnung 14. Diese hat Rast-Zahnreihen 15 mit hintereinander längs der mittleren Teleskophülse 11 und der inneren Verbindungs-Teleskophülse 12 angeordneten Rastzähnen 16. Jede der Teleskophülsen 11, 12 hat zwei in Umfangsrichtung um die Längsachse der Injektionsvorrichtung einander gegenüberliegend angeordnete äußere Rast-Zahnreihen 15. Die beiden Rast-Zahnreihen 15 der mittleren Teleskophülse 11 sind relativ zu den beiden Zahnreihen 15 der inneren Verbindungs-Teleskophülse 12 um 90° in Umfangsrichtung um die Längsachse der Injektionsvorrichtung 1 versetzt.

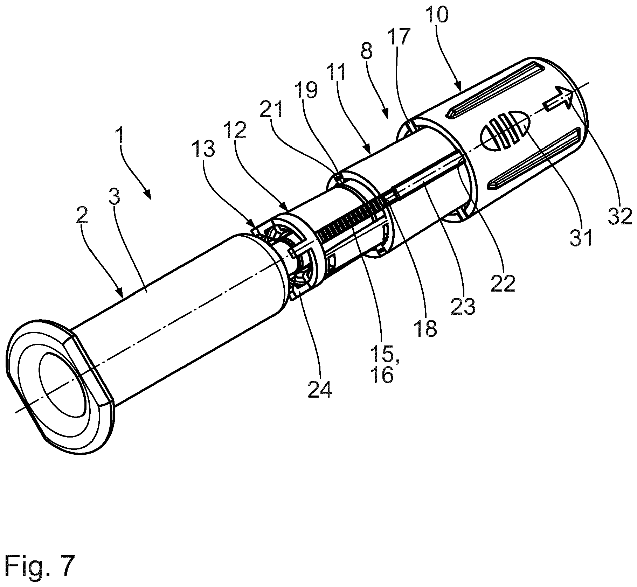

In die Rastzähne 16 greift jeweils ein Gegenrastkörper 17 der äußeren Schutz-Teleskophülse 10 bzw. ein Gegenrastkörper 18 (vgl. Fig. 7) der mittleren Teleskophülse 11 ein. Die Rastzähne 16 haben im axialen Längsschnitt der Injektionsvorrichtung ein Sägezahnprofil mit einer Vorzugsrichtung, um eine Einweg-Umstellung der Teleskophülsen 10, 11 von der Injektionsstellung in die teleskopierte Schutzstellung zu gewährleisten. Ein Rastzahn 16' (vgl. Fig. 6), der einer maximal teleskopierten Relativstellung der zugeordneten Teleskophülsen 10, 11 entspricht, hat im Axialschnitt eine genau gegenläufige Vorzugsrichtung, um die End-Teleskopierstellung, also die Schutzstellung der Stechschutzeinrichtung 8 zu definieren.

Die Rastzähne 16 sind einstückig an die jeweilige Teleskophülse 11, 12 angeformt.

Zur Gewährleistung einer Teleskop-Führung und gleichzeitig eines Verdrehschutzes zwischen zwei benachbarten der drei Teleskophülsen 10 bis 12, also zwischen den Teleskophülsen 10, 11 einerseits und den Teleskophülsen 11, 12 andererseits dienen Nut/Feder-Führungseinrichtungen 19 der Stechschutzeinrichtung 8.

Fig. 7 zeigt eine Nut 20 einer der Nut/Feder-Führungseinrichtungen 19, die in einer äußeren Mantelwand der inneren Verbindungs-Teleskophülse 12 als axiale Längsnut ausgeführt ist. In diese Nut 20 greift eine komplementär ausgeführte Feder 21 ein, die in einer Innenwand der mittleren Teleskophülse 11 nach innen vorstehend ausgeführt ist. Die Feder 21 der mittleren Teleskophülse 11, die mit der Nut 20 der inneren Teleskophülse 12 führend zusammenwirkt, ist durch die inneren Enden der Restzähne 16 der mittleren Teleskophülse 11 gebildet.

Eine weitere Nut/Feder-Führungseinrichtung 19 ist gebildet durch axiale Längsnuten 22 in der inneren Mantelwand der äußeren Schutz-Teleskophülse 10 und hierzu komplementäre Federn 23, die radial nach außen überstehend in der äußeren Mantelwand der mittleren Teleskophülse 11 ausgeführt sind. Jeweils zwei gleichartige Nut/Feder-Führungseinrichtungen 19 sind einander gegenüberliegend in Bezug auf die Längsachse der Injektionsvorrichtung 1 angeordnet. Jeweils bezogen auf eine der Teleskophülsen 10 bis 12 wechselt sich in Umfangrichtung um die Längsachse der Injektionsvorrichtung 1 jeweils in 90°-Schritten eine Rastkomponente der Schutz-Rastanordnung 14 mit einer Komponente der Nut/Feder-Führungseinrichtung 19 ab.

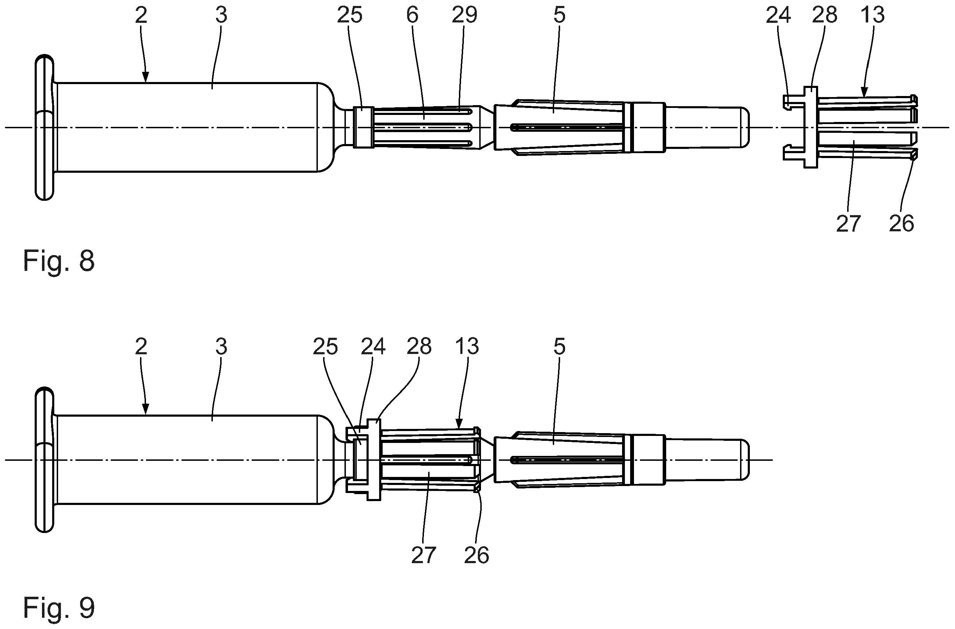

Die Fig. 8 bis 11 zeigen Momentanpositionen bei der Montage der Injektionsvorrichtung 1. Der Formschluss-Adapter 13 ist mithilfe von Rasthaken 24 rastend mit dem Behälter 3 der Injektionseinrichtung 2 verbunden. Hierzu hintergreifen die Rasthaken 24 einen Rastbund 25 des Behälters 3, der am Übergang zum Mündungsabschnitt 6 angeordnet ist.

Die innere Verbindungs-Teleskophülse 12 ist axial mit dem Formschluss-Adapter 13 über eine Mehrzahl von Rastkörpern 26 verbunden, die an freien Enden von Rastzungen 27 des Formschluss-Adapters 13 angeformt sind. Die Rastzungen 27 erstrecken sich in axialer Richtung und sind an einen gemeinsamen Tragring 28 des Formschluss-Adapters 13 angeformt. Insgesamt erhält hierdurch der Formschluss-Adapter 13 die Form einer axial aufsteckbaren Adapterhülse. Ein Abstand zwischen zwei in Umfangrichtung um die Längsachse der Injektionsvorrichtung 1 benachbarten Rastzungen 27 und die Anzahl der Rastzungen 27 ist abgestimmt auf eine Breite und eine Anzahl von axial verlaufenden Umfangsrippen 29, die außen an den Mündungsabschnitt 6 des Behälters 3 angeformt sind. Jeweils eine der Rastzungen 27 fügt sich bei aufgestecktem Formschluss-Adapter 13 zwischen zwei Benachbarten der Umfangsrippen 29, sodass ein Verdrehschutz des Formschluss-Adapters 13 relativ zum Behälter 3, nämlich zum Mündungs- und Verbindungsabschnitt 6 des Behälters 3,gegeben ist. Eine Innenwand der inneren Verbindungs-Teleskophülse 12 ist mit Axialstrukturen versehen, die in der Zeichnung nicht näher dargestellt sind und die bei auf den Formschluss-Adapter 13 aufgerasteter innerer Verbindungs-Teleskophülse 12 für einen Verdrehschutz der inneren Verbindungs-Teleskophülse 12 relativ zum Formschluss-Adapter 13 sorgen. Die inneren Axialstrukturen der Verbindungs-Teleskophülse 12 greifen dabei zwischen jeweils benachbarten Rastzungen 27 des Formschluss-Adapters 13 ein.

Bei aufgerasteter innerer Verbindungs-Teleskophülse 12 hintergreifen die Rastkörper 26 einen komplementär hierzu gebildeten Rastbund der Verbindungs-Teleskophülse 12, was in der Zeichnung nicht näher dargestellt ist.

Zum formschlüssigen Festlegen der Schutz-Teleskophülse 10, also der Schutzkomponente der Stechschutzeinrichtung 8, an der Injektionseinrichtung 2 in der Injektionsstellung dient eine Injektions-Verbindungsanordnung, die als Injektions-Rastanordnung ausgeführt ist. Rastkomponenten dieser Injektions-Rastanordnung sind einerseits die äußeren Kanten der freien Enden der Rasthaken 24 des Formschluss-Adapters 13 und andererseits die in der Injektionsstellung diese hintergreifenden Gegenrastkörper 17 der Schutz-Teleskophülse 10. Diese Injektions-Rastanordnung 17, 24 ist durch Ausklinken der Gegenrastkörper 17 aus dem Hintergriff zu den Rastzungen 24 überwindbar. Diese Überwindung kann durch einen definierten Druck auf die Stechschutzeinrichtung 8 erreicht werden.

Die Injektionsvorrichtung 1 wird folgendermaßen montiert: Zunächst liegt die Injektionseinrichtung 2 in einer handelsüblichen Auslieferungs-Gestaltung vor, die in der Fig. 8 wiedergegeben ist. Die Stechschutzeinrichtung 8 mit den Hülsen 10 bis 12 ist in der Injektionsstellung vormontiert, in der die Teleskophülsen vollständig übereinandergeschoben sind. Auf die Injektionseinrichtung 2 wird dann der Formschluss-Adapter 13 von der Kanülenseite der Injektionseinrichtung 2 her mit führenden Rasthaken 24 aufgeschoben, bis die Rasthaken 24 den Rastbund 25 des Behälters 3 rastend hintergreifen (vgl. Fig. 9). Anschließend wird die vorgefertigte Stechschutzeinrichtung 8 mit den drei ineinander gesteckten und rastend miteinander verbundenen Teleskophülsen 10 bis 12, ebenfalls von der Kanülenseite der Injektionseinrichtung 2 her auf diese aufgeschoben, bis einerseits die innere Verbindungs-Teleskophülse 12 auf den Formschluss-Adapter 13 aufgerastet ist, wobei die Rastzungen 27 radial zwischen die Umfangsrippen 29 zum Verdrehschutz gedrückt werden, und andererseits die Injektions-Rastanordnung 17, 24 zum Verrasten kommt. Die innere Verbindungs-Teleskophülse 12 wird in Umfangsrichtung so orientiert auf den Formschluss-Adapter 13 aufgeschoben, dass die inneren Axialstrukturen der Verbindungs-Teleskophülse 12 zwischen die Rastzungen 27 des Formschluss-Adapters 13 eingreifen. Bei vollständig aufgeschobener innerer Verbindungs-Teleskophülse 12 kommt ein führender Anschlagsbund der Verbindungs-Teleskophülse 12 in Anschlag an eine zugewandte Stirnwand des Tragrings 28 des Formschluss-Adapters 13.

Innere Strukturen der Verbindungs-Teleskophülse 12 dienen gleichzeitig als Niederhaltemittel zum Halten der Rastzungen 27 zwischen den Umfangsrippen 29 des Mündungsabschnitts 6 der Injektionseinrichtung 2.

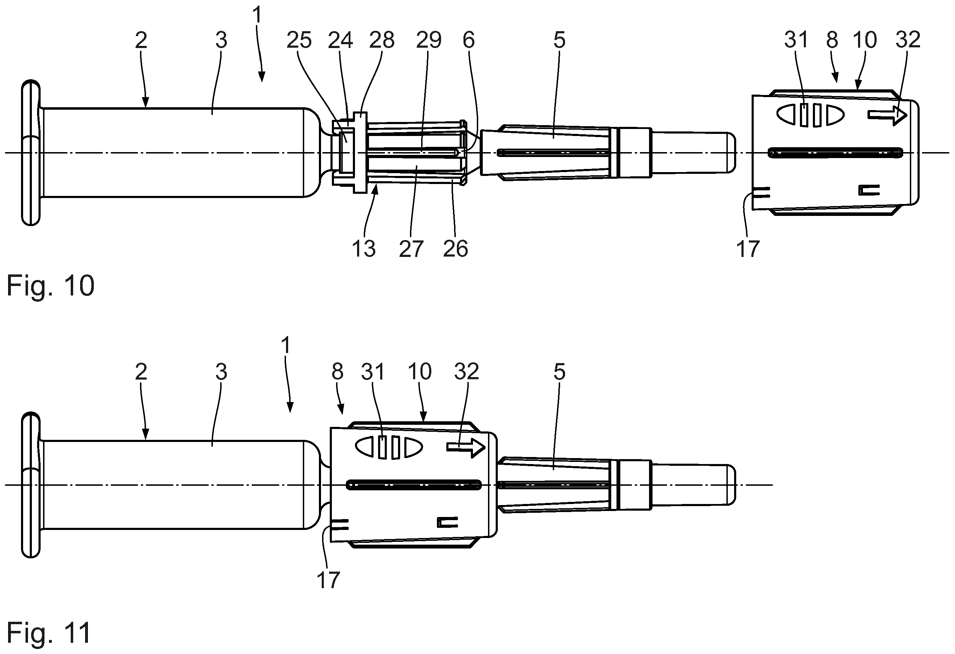

Nach der Montage liegt die Injektionsvorrichtung 1 mit der Stechschutzeinrichtung 8 in der Injektionsstellung und der ursprünglich schon montierten Schutzkappe 5 über der Injektionsnadel 4 vor, wie in den Fig. 1 und 11 dargestellt. Durch die verschiedenen Verdrehschutzkomponenten sind die insgesamt vier Bauteile 10 bis 13 der Stechschutzeinrichtung 8 zueinander verdrehgesichert und auch die gesamte Stechschutzeinrichtung 8 ist zur Injektionseinrichtung 2 verdrehgesichert.

Die Injektionsvorrichtung 1 wird folgendermaßen benutzt: Zunächst wird die Schutzkappe 5 von der Injektionskanüle 4 entfernt, was durch eine Abdrehbewegung geschieht (vgl. Pfeil 30 in der Fig. 1). Beim Abdrehen ist durch die über den Außenumfang des Behälters 3 überstehende Querschnittsgestaltung der Schutz-Teleskophülse 10 gesichert, dass der Benutzer zum Abdrehen der Schutzkappe 5 die Injektionsvorrichtung 1 außen an der Schutz-Teleskophülse 10 ergreift. Hierzu hat die Schutz-Teleskophülse 10 axial verlaufende Längsrippen, die verhindern, dass sich beim Abdrehen der Schutzkappe 5 die Schutz-Teleskophülse 10 unerwünscht zwischen den Fingern des Benutzers dreht. Da alle Bauteile der Stechschutzeinrichtung 8 zueinander und auch der Formschluss-Adapter 13 zum Mündungsabschnitt 6 verdrehgesichert sind, ist bei einer Relativverdrehung der Schutzkappe 5 gegen die Stechschutzeinrichtung 8 in Richtung des Pfeils 30 (oder in Gegenrichtung hierzu) gewährleistet, dass sich die Schutzkappe 5 tatsächlich gewünscht vom Mündungsabschnitt 6 abdreht. Nach diesem Abdrehen kann die Schutzkappe 5 von der Injektionskanäle 4 abgezogen werden.

Die Injektionsvorrichtung 1 liegt dann im gebrauchsfertigen Zustand vor, der in der Fig. 2 dargestellt ist. Zum Verlagern der Stechschutzeinrichtung 8 in die Schutzstellung (vgl. hierzu die Fig. 3 bis 7) wird zunächst in einem Druckbereich 31, der auf der äußeren Schutz-Teleskophülse 10 markiert ist, von beiden Seiten her Druck auf die Schutz-Teleskophülse 10 ausgeübt. Hierdurch klinken die Gegenrastkörper 17 von den Rasthaken 24 aus und die äußere Schutz-Teleskophülse 10 kann in Richtung des dort aufgebrachten Pfeils 32 axial relativ zu der Teleskophülse 11 teleskopiert werden (vgl. auch Pfeil 33a in der Fig. 3). Dabei rattert der Gegenrastkörper 17 über die Rastzähne 16 der Schutz-Rastanordnung 14, bis die Endstellung des Gegenrastkörpers 17 vor dem endseitigen Rastzahn 16' der mittleren Teleskophülse 11 erreicht ist. Anschließend teleskopiert auch die mittlere Teleskophülse 11 relativ zur inneren Verbindungs-Teleskophülse 12, wobei die Gegenrastkörper 18 der mittleren Teleskophülse 11 über die Rastzähne 16 der inneren Verbindungs-Teleskophülse 12 rattern, bis auch hier die Endstellung des Gegenrastkörpers 18 in Anlage wiederum zum endseitigen Rastzahn der inneren Verbindungs-Teleskophülse 12 erreicht ist. Die Stechschutzeinrichtung 8 liegt dann in der komplett teleskopierten Schutzstellung nach Fig. 4 vor. In dieser ist die Kanülenspitze 9 der Injektionskanüle 4 in der Schutz-Teleskophülse 10 vollständig versenkt angeordnet, sodass ein sicherer Stechschutz gewährleistet ist. Eine zerstörungsfreie Überführung der Stechschutzeinrichtung 8, ausgehend von der Schutzstellung nach Fig. 4, sodass die Kanülenspitze 9 wieder frei wird, ist für den Benutzer aufgrund der Einweg-Charakteristik der zugeordneten Rastanordnungen 14 nicht möglich.

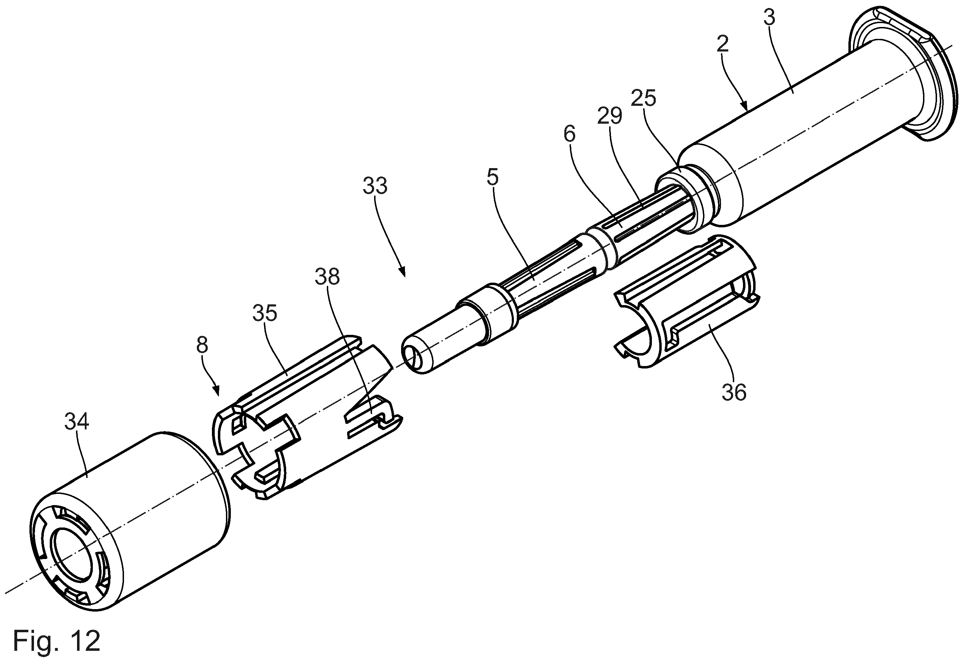

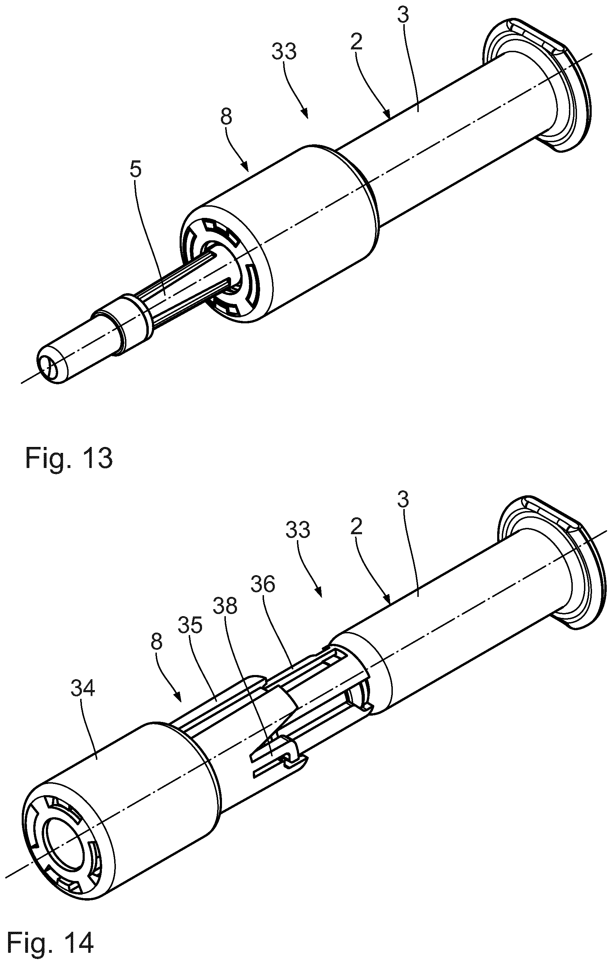

Anhand der Fig. 12 bis 17 wird nachfolgend eine weitere Ausführung einer Injektionsvorrichtung 33 erläutert. Komponenten und Funktionen, die denjenigen entsprechen, die vorstehend unter Bezugnahme auf die Fig. 1 bis 11 bereits beschrieben wurden, tragen die gleichen Bezugsziffern und werden nicht nochmals im Einzelnen diskutiert.

Die Stechschutzeinrichtung 8 der Injektionsvorrichtung 33 nach den Fig. 12 bis 17 hat wiederum drei Teleskophülsen, nämlich eine äußere Schutz-Teleskophülse 34, deren Funktion der Schutz-Teleskophülse 10 der Ausführung nach den Fig. 1 bis 11 entspricht, eine mittlere Teleskophülse 35, deren Funktion der Teleskophülse 11 der Ausführung nach den Fig. 1 bis 11 entspricht, und eine innere Verbindungs-Teleskopkomponente 36, die gleichzeitig einen Formschluss-Adapter zur Formschlussverbindung der Stechschutzeinrichtung 8 mit der Injektionseinrichtung 2 darstellt. Die Verbindungs-Teleskopkomponente 36 vereint also die Funktionen der inneren Teleskophülse 12 und des Formschluss-Adapters 13 der Ausführung nach den Fig. 1 bis 11.

Die Verbindungs-Teleskopkomponente 36 ist als radial aufrastbarer, C-förmiger Adapter ausgeführt. Die Verbindungs-Teleskopkomponente 36 wird radial auf den Mündungsabschnitt 6 des Behälters 3 aufgerastet, wobei zur axialen Sicherung der Verbindungs-Teleskopkomponente 36 der Rastbund 25 des Mündungsabschnitts 6 in einem Umfangsbereich von der Verbindungs-Teleskopkomponente 36 hintergriffen wird.

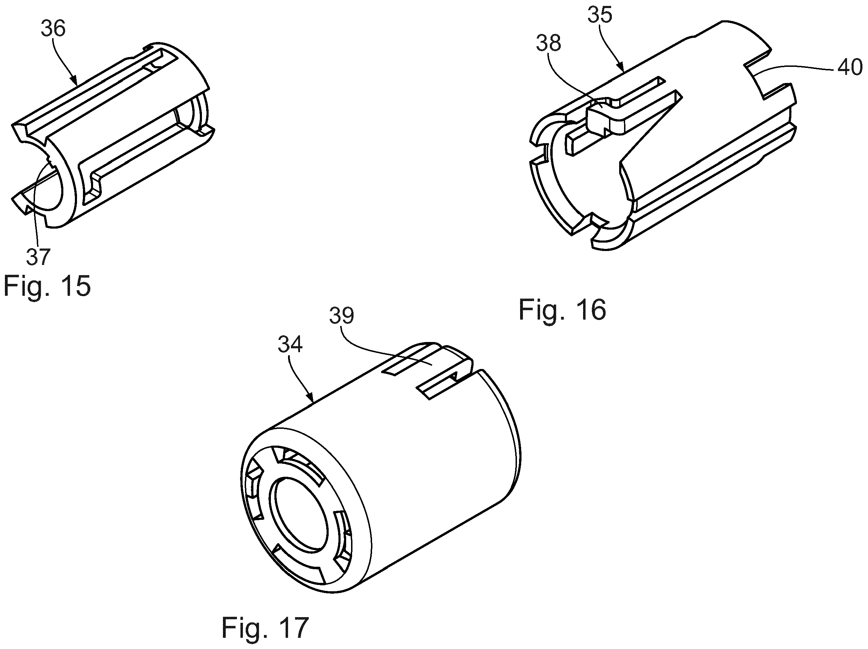

Um einen höheren Reibschluss zwischen der Verbindungs-Teleskopkomponente 36 und dem Mündungsabschnitt 6 des Behälters 3 der Injektionseinrichtung 2 und damit insbesondere einen Verdrehschutz zu gewährleisten, sind innere Rippen 37 der Verbindungs-Teleskopkomponente 36, die in montiertem Zustand mit dem Mündungsabschnitt 6 zwischen dessen Umfangsrippen 29 zur Anlage kommen, aus weicherem Kunststoffmaterial ausgeführt als der sonstige Grundkörper der Verbindungs-Teleskopkomponente 36. Die Rippen 37 können an den Grundkörper der Verbindungs-Teleskopkomponente 36 beispielsweise durch Mehrkomponenten-Technik, insbesondere durch 2K-Technik, angeformt sein. Von den inneren Rippen 37 ist in der Fig. 15 genau eine Rippe 37 angedeutet. Tatsächlich liegen mehrere, z. B. fünf, Rippen 37 in Umfangsrichtung abgestimmt auf die Umfangsabstand der Umfangsrippen 29 gleichbeabstandet vor.

Die mittlere Teleskophülse 35 (vgl. auch Fig. 16) ist mit der Verbindungs-Teleskopkomponente 36 über eine radial wirkende Rastverbindung verbunden. Hierzu hat die mittlere Teleskophülse 35 eine Federzunge 38, die in eine entsprechende Rastausnehmung der Verbindungs-Teleskopkomponente 36 eingreift.

Die äußere Schutz-Teleskophülse 34 hat ebenfalls eine Federzunge 39, die zur rastenden Verbindung eine entsprechende Rastaufnahme in der mittleren Teleskophülse 35 bzw. der Verbindungs-Teleskopanordnung 36 hintergreift. Die Federzunge 39 und die in der Injektionsstellung zugeordnete Rastaufnahme bilden somit die Injektions-Verbindungsanordnung zum formschlüssigen Festlegen der Teleskop-Schutzhülse 34 an der Injektionseinrichtung 2 in der Injektionsstellung.

In der Schutzstellung der Stechschutzeinrichtung 8 in der Ausführung nach den Fig. 12 bis 17 hintergreift die Rastzunge 39 eine Rastaufnahme 40, die in der mittleren Teleskophülse 35 ausgeführt ist. Hierdurch und durch eine entsprechende Rastverbindung der mittleren Teleskophülse 35 an der Verbindungs-Teleskopkomponente 36 ist eine Schutz-Rastanordnung zum rastenden Festlegen der Schutz-Teleskophülse 34 in der Schutzstellung gegeben.

Abgesehen von den vorstehend erläuterten Unterschieden entspricht die Montage und auch die Benutzung der Injektionsvorrichtung 33 dem, was vorstehend unter Bezugnahme auf die Injektionsvorrichtung 1 bereits erläutert wurde.

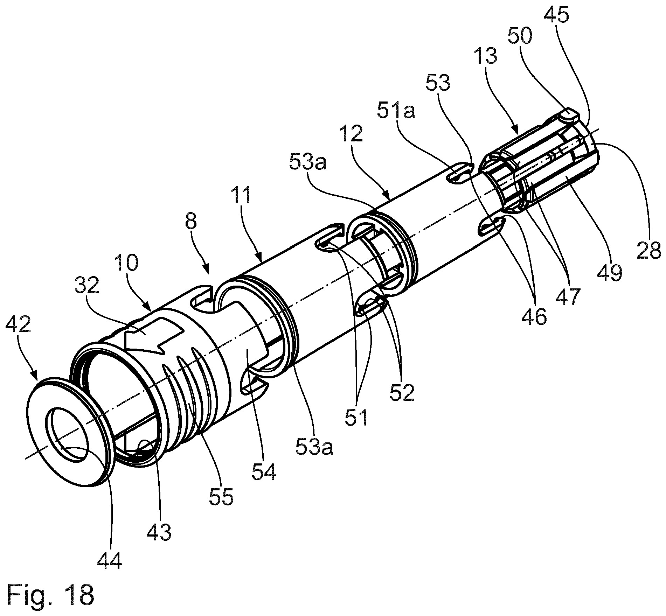

Anhand der Fig. 18 bis 26 wird nachfolgend eine weitere Ausführung einer Injektionsvorrichtung 41 erläutert. Komponenten und Funktionen, die denjenigen entsprechen, die vorstehend unter Bezugnahme auf die Injektionsvorrichtungen 1 und 33 bereits erläutert wurden, tragen die gleichen Bezugsziffern und werden nicht nochmals im Einzelnen diskutiert.

Die Injektionsvorrichtung 41 hat ebenfalls eine Teleskop-Stechschutzeinrichtung 8, die mit einer Verbindungs-Teleskophülse 12, einem Formschluss-Adapters 13, einer mittleren Teleskophülse 11 und einer Schutz-Teleskophülse 10 grundsätzlich so aufgebaut ist wie die Schutzeinrichtung 8 der Injektionsvorrichtung 1. Unterschiede ergeben sich in Details der Rastverbindungen und der Führungsgestaltungen. Die Rastverbindungen sind bei der Injektionsvorrichtung 41 als Axial-Rastverbindungen ausgeführt.

Die Schutz-Teleskophülse 10 ist bei der Stechschutzeinrichtung 8 der Injektionsvorrichtung 41 zweiteilig ausgeführt und hat neben der eigentlichen Teleskophülse noch einen ringförmigen Deckel 42. Über einen Außenumfang ist der Deckel 42 in eine innere Umfangsnut 43 in einem äußeren Endbereich der Schutz-Teleskophülse 10 eingerastet. Durch einen äußeren Umfang des Deckels 42 und die innere Umfangsnut 43 ist eine Ringschnappverbindung zwischen dem Deckel 42 und der Schutz-Teleskophülse 10 gebildet. Der Deckel 42 dient dazu, eine von außen zugängliche Öffnungsweite der Schutz-Teleskophülse 10 auf eine Durchgangsöffnung 44 mit im Vergleich zum Innendurchmesser der sonstigen Schutz-Teleskophülse 10 verringertem Durchmesser zu reduzieren.

Bei der Montage der Stechschutzeinrichtung 8 nach den Fig. 18 bis 26 wird zunächst die mittlere Teleskophülse 11 von der Seite der inneren Umfangsnut 43 her in die noch ohne Deckel vorliegende Schutz-Teleskophülse 10 eingesetzt. Anschließend wird von der gleichen Seite her die Verbindungs-Teleskophülse 12 in die mittlere Teleskophülse 11 eingesetzt. Sodann wird der Deckel 42 in die innere Umfangsnut 43 eingerastet. Von der gegenüberliegenden Seite wird dann der Formschluss-Adapter 13 in die Verbindungs-Teleskophülse 12 eingesetzt.

Die so vormontierte Stechschutzeinrichtung kann dann auf die Injektionseinrichtung 2 aufgeschoben werden. Dies geschieht, bis ein Anlagebund 45 des Formschluss-Adapters 13 am Rastbund 25 der Injektionseinrichtung 2 (vgl. z. B. Fig. 8) anliegt.

Beim weiteren Aufschieben der Stechschutzeinrichtung 8 nach den Fig. 18 bis 26 auf die Injektionseinrichtung 2 in Richtung auf den Behälter 3 zu wird die Verbindungs-Teleskophülse 12 axial zum dann axial am Rastbund 25 festgelegten Formschluss-Adapter 13 verschoben, bis Rasthaken 46, die an die Verbindungs-Teleskophülse 12 angeformt sind, den Rastbund 25 des Mündungsabschnitts 6 der Injektionseinrichtung 2 hintergreifen. Bei der Injektionsvorrichtung 41 dienen also nicht Rasthaken am Formschluss-Adapter 13, sondern die Rasthaken 46 an der Verbindungs-Teleskophülse 12 zum formschlüssigen Festlegen unter anderem der Schutz-Teleskophülse 10 als Schutzkomponente der Stechschutzeinrichtung 8 an der Injektionseinrichtung 2 in der Injektionsstellung.

Auch bei der Injektionsvorrichtung 41 ist ein Verdrehschutz zwischen der Stechschutzeinrichtung 8, die also auch eine Verdrehschutzeinrichtung ist, und dem Mündungsabschnitt 6 der Injektionseinrichtung 2 gegeben. Hierzu hat der Formschluss-Adapter 13 der Injektionsvorrichtung 41 wiederum Verdrehschutz-Zungen 47, die den Rastzungen 27 der Ausführung nach den Fig. 1 bis 11 entsprechen. Die Verdrehschutz-Zungen 47 verlaufen axial und sind über den Tragring 28 des Formschluss-Adapters 13 der Injektionsvorrichtung 41 miteinander verbunden. Montiert sind die Verdrehschutz-Zungen 47 jeweils zwischen zwei benachbarten, axial verlaufenden Umfangsrippen 29 des Mündungsabschnitts 6 der Injektionseinrichtung 2 aufgenommen.

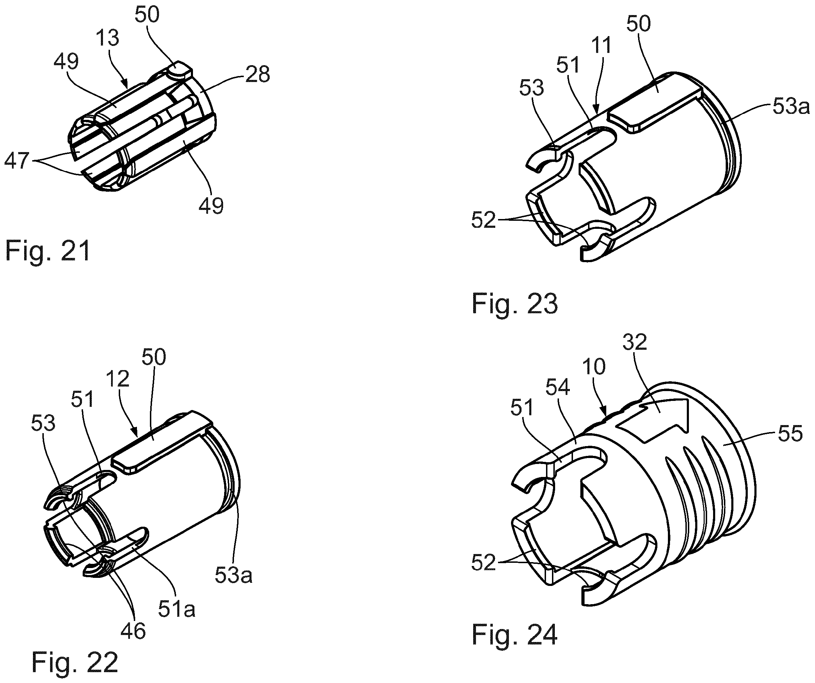

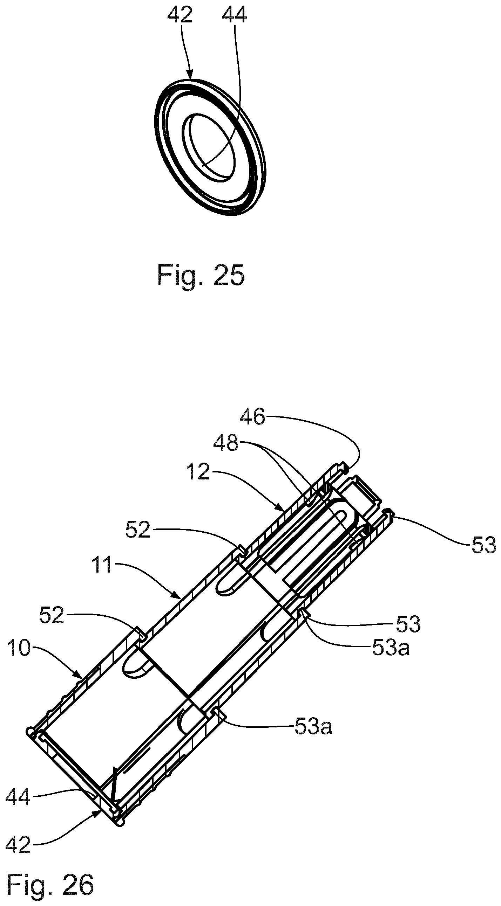

Zum Halten der Verdrehschutz-Zungen 47 zwischen den Umfangsrippen 29 dient ein an der Verbindungs-Teleskophülse 12 ausgebildetes Niederhaltemittel. Dieses ist gebildet durch insgesamt vier innere Axialrippen, von denen zwei im Axialschnitt nach Fig. 26 sichtbar sind. Die Axialrippen 48 sind jeweils um 90° in Umfangsrichtung versetzt an einer Innenwand der Verbindungs-Teleskophülse 12 angeformt. Jede der Axialrippen 48 wirkt mit einem Niederhalte-Gegenstück am Formschluss-Adapter 13 zum Niederhalten jeweils einer Verdrehschutz-Zunge 47 zusammen. Die Niederhalte-Gegenstücke sind durch äußere Axialrippen 49 am Formschluss-Adapter 13 ausgebildet (vgl. Fig. 21).

Der Formschluss-Adapter 13 ist an der Verbindungs-Teleskophülse 12 und jeweils zwei aneinander anliegende Teleskophülsen 12, 11, 10 sind gegeneinander jeweils durch Verdrehsicherungen gegen eine Relativverdrehung um die Längsachse der Stechschutzeinrichtung 8 gesichert. Diese Verdrehsicherung ist wiederum gebildet durch äußere Federn 50 an jeweils einer der Komponenten 13, 12, 11, die mit hierzu komplementären inneren Axialnuten 51 in den jeweils benachbarten, außenanliegenden Teleskophülsen 12, 11, 10 verdrehsichernd zusammenwirken.

Die Federn 50 dienen gleichzeitig als Anschläge, die mit axial verlaufenden Ausnehmungen 51a als Anschläge zur Vorgabe einer axialen Endposition der Verbindungs-Teleskophülse 12 relativ zum Formschluss-Adapter 13 bei der rastenden Verbindung der Stechschutzeinrichtung 8 mit dem Rastbund 25 des Mündungsabschnitts 6 über die Rasthaken 46 der Verbindungs-Teleskophülse 12 zusammenwirken.

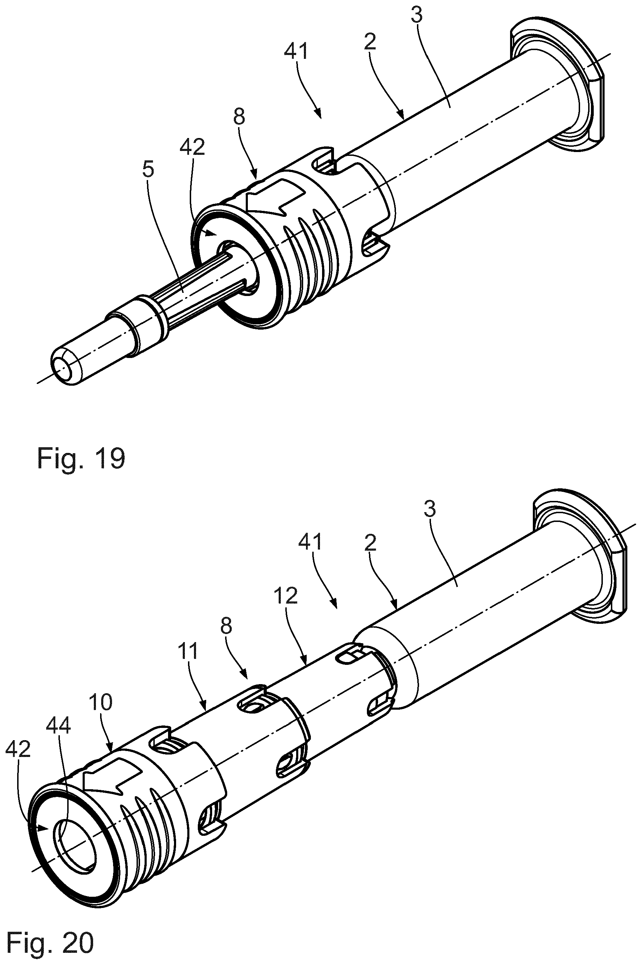

Vergleichbar zu den Rasthaken 46 der Verbindungs-Teleskophülse 12 haben auch die mittlere Teleskophülse 11 und die Schutz-Teleskophülse 10 vergleichbare, radial wirkende Rasthaken 52. Genau wie die Rasthaken 46 sind auch die Rasthaken 52 in Umfangsrichtung jeweils um 90° versetzt zueinander angeordnet. In der Injektionsstellung, beispielsweise nach Fig. 19, liegen die Rasthaken 46, 52 benachbarter Teleskophülsen 12, 11, 10 genau übereinander. Die Rasthaken 52 der mittleren Teleskophülse 11 hintergreifen dabei hierzu komplementäre Ausnehmungen 53 in der Außenseite der Rasthaken 46. Die Rasthaken 52 der Schutz-Teleskophülse 10 hintergreifen entsprechende Ausnehmungen 53 in der Außenseite der Rasthaken 52 der mittleren Teleskophülse 11.

Die Rasthaken 52 der mittleren Teleskophülse 11 einerseits und der Schutz-Teleskophülse 10 andererseits wirken in der Schutzstellung der Stechschutzeinrichtung 8 (vgl. z. B. Fig. 20 und 26) mit äußerem Umfangsnuten 53a einerseits in der Verbindungs-Teleskophülse 12 und andererseits in der mittleren Teleskophülse 11 zusammen.

Bei der Überführung der Teleskophülsen 11, 10 von der zusammengeschobenen Injektionsstellung in die ausgefahrene Schutzstellung gleiten die Rasthaken 52 zwischen den jeweiligen Gegen-Ausnehmungen 53 und den Umfangsnuten 53a. Damit bei der Überführung der Teleskophülsen 11, 10 in die Schutzstellung eine gleichmäßige Kraftentfaltung auf die Rasthaken 52 erfolgt, erweitern sich die Teleskophülsen 12, 11 zwischen den jeweiligen Gegenausnehmungen 53 und den jeweiligen Umfangsnuten 53a konisch.

Die Schutz-Teleskophülse 10 ist als 2K-Spritzgussteil ausgeführt. Neben einem Tragkörper 54 hat die Schutz-Teleskophülse 10 einen Griffabschnitt 55. Der Tragkörper 54 einerseits und der Griffabschnitt 55 andererseits sind als unterschiedliche Spritzguss-Komponenten des 2K-Bauteils ausgeführt. Als 2K-Kunststoffe können ABS (Acrylnitril-Butadien-Styrol) für eine Hartkomponente, also beispielsweise für den Tragkörper 54, und TPE (thermoplastisches Elastomer) für eine Weichkomponente, beispielsweise für den Quickabschnitt 55, zum Einsatz kommen. Auch eine andere Anzahl von Komponenten können bei einem solchen Mehrkomponenten-Spritzgussteil zum Einsatz kommen, beispielsweise drei oder mehr Komponenten aus verschiedenen und insbesondere aus verschieden harten Kunststoffen.

Durch die Ausbildung der Schutz-Teleskophülse 10 als 2K-Spritzgussteil ist im Bereich des Griffabschnitts 55 eine griffigere Haptik der Schutz-Teleskophülse 10 gewährleistet.

Auch die als Niederhaltemittel dienenden Axialrippen 48 der Verbindungs-Teleskophülse 12 können aus einem zur sonstigen Verbindungs-Teleskophülse 12 anderem Kunststoffmaterial gefertigt sein und die Axialrippen 48 können mittels 2K-Spritzgustechnik an einen sonstigen Tragkörper der Verbindungs-Teleskophülse 12 angeformt sein.

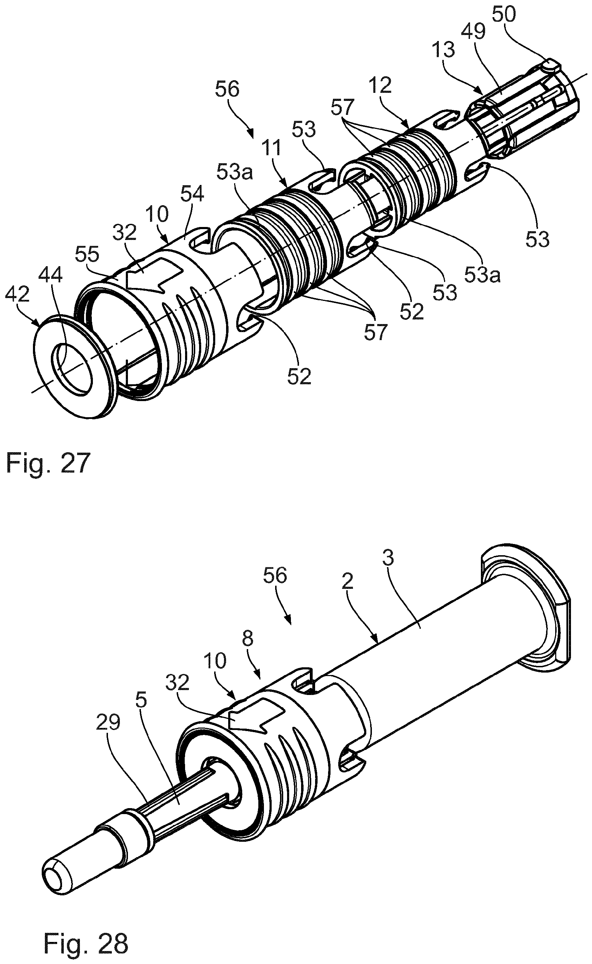

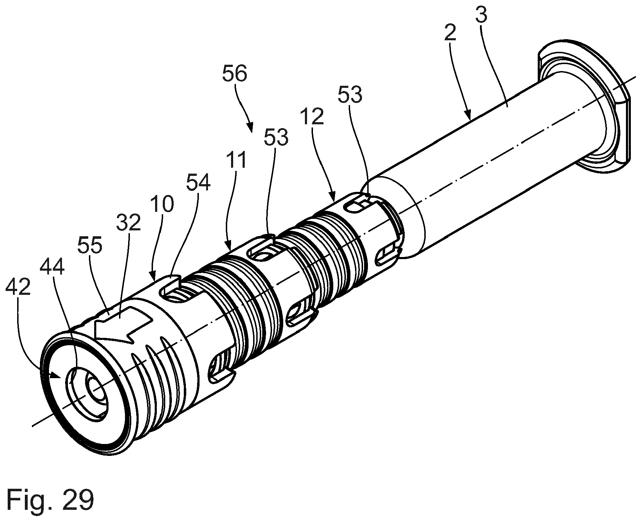

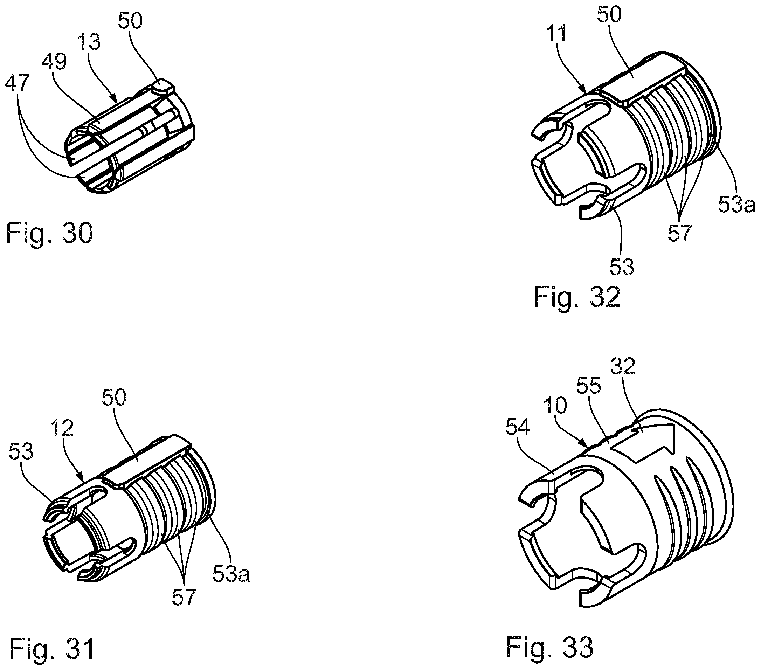

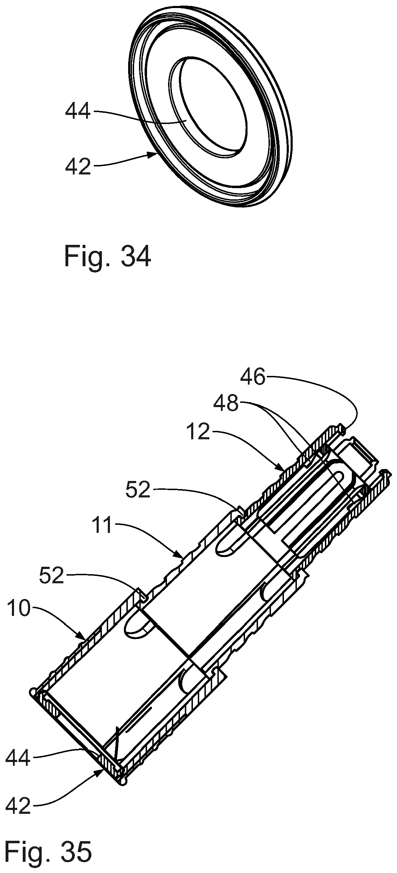

Anhand der Fig. 27 bis 35 wird nachfolgend eine weitere Ausführung einer Injektionsvorrichtung 56 erläutert. Komponenten und Funktionen, die denjenigen entsprechen, die vorstehend unter Bezugnahme auf die Injektionsvorrichtungen 1, 33 und 41 und insbesondere unter Bezugnahme auf die Injektionsvorrichtung 41 bereits erläutert wurden, tragen die gleichen Bezugsziffern und werden nicht nochmals im Einzelnen diskutiert.

Zwischen den Gegenausnehmungen 53 und den Umfangsnuten 53a haben die Verbindungs-Teleskophülse 12 und die mittlere Teleskophülse 11 jeweils drei Rast-Zwischenstufen 57. Beim Verlagern der Schutz-Teleskophülse 10 und der mittleren Teleskophülse 11 zwischen der Injektionsstellung und der Schutzstellung rasten die jeweiligen Rasthaken 52 der Schutz-Teleskophülse 10 und der mittleren Teleskophülse 11 auf ihrem Verlagerungsweg zwischen den jeweiligen Gegenausnehmungen 53 und den jeweiligen Umfangsnuten 53a über die Rast-Zwischenstufen 57. Es ergibt sich auf dem Weg der beiden Teleskophülsen 10, 11 zwischen der Injektionsstellung und der Schutzstellung eine haptische Rückmeldung für den Benutzer hinsichtlich des bereits zurückgelegten Verstellweges.

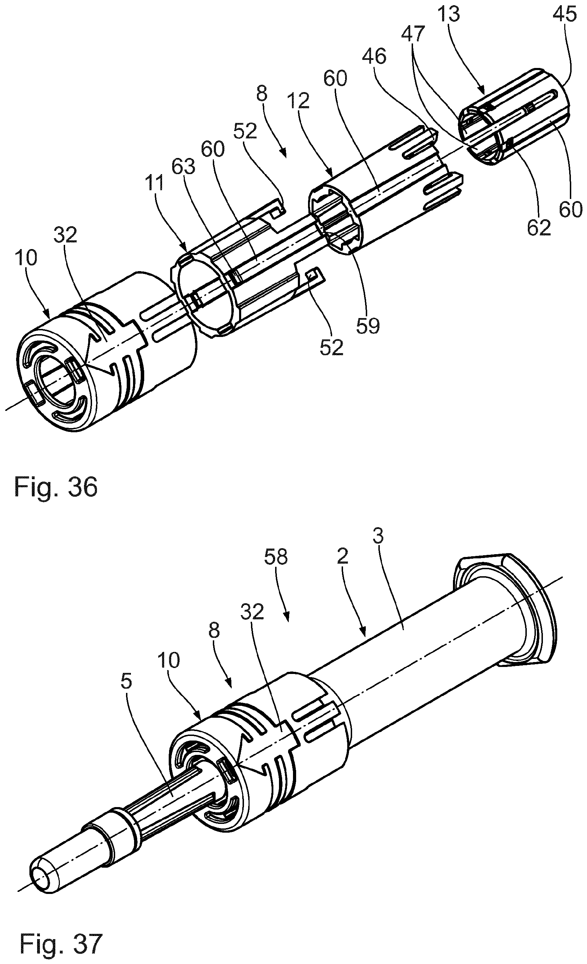

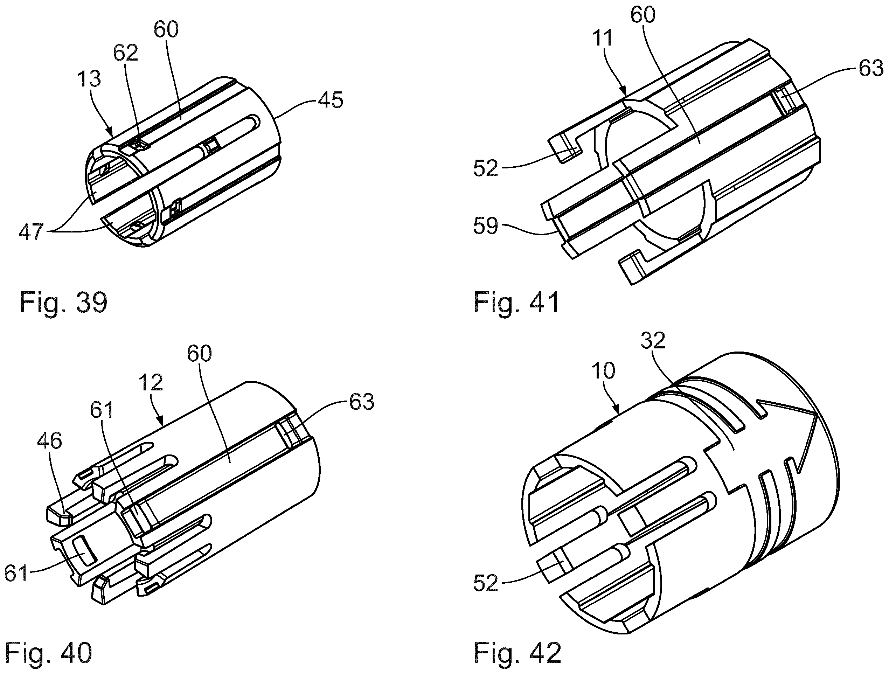

Anhand der Fig. 36 bis 43 wird nachfolgend eine weitere Ausführung einer Injektionsvorrichtung 58 erläutert. Komponenten und Funktionen, die denjenigen entsprechen, die vorstehend unter Bezugnahme auf die Injektionsvorrichtungen 1, 33, 41 und 56 bereits erläutert wurden, tragen die gleichen Bezugsziffern und werden nicht nochmals im Einzelnen diskutiert.

Auch die Injektionsvorrichtung 58 ist, ähnlich wie die Injektionsvorrichtung 1, vierteilig ausgeführt mit einem inneren Formschluss-Adapter 13, einer Verbindungs-Teleskophülse 12, einer mittleren Teleskophülse 11 und einer äußeren Schutz-Teleskophülse 10.

Die Funktion von Rasthaken 46 und 52 der Teleskophülsen 12, 11 und 10 bei der Injektionsvorrichtung 58 ähnelt der Injektionsvorrichtung 41. Die Teleskophülsen 11 und 10 haben jeweils zwei einander gegenüberliegende, in Umfangsrichtung also um 180° zueinander versetzt angeordnete Rasthaken 52. Bei montierter Injektionsvorrichtung 8 sind die Rasthaken 52 der mittleren Teleskophülse 11 relativ zu den Rasthaken 52 der Schutz-Teleskophülse 10 um 90° in Umfangsrichtung versetzt, ähnlich wie beim Konzept der Gegenrastkörper und der Rastzähne bei der Injektionsvorrichtung 1.

Bei der Montage wird zunächst die mittlere Teleskophülse 11 in die äußere Schutz-Teleskophülse 10 in Richtung des Pfeils 32 eingeschoben, bis die Rasthaken 52 der äußeren Schutz-Teleskophülse 10 Gegenausnehmungen 59 der mittleren Teleskophülse 11 hintergreifen, die am Ende von axialen Führungsbahnen 60 in einer Außenwand der mittleren Teleskophülse 11 ausgeführt sind.

Anschließend wird die Verbindungs-Teleskophülse 12 ebenfalls in Richtung des Pfeils 32 in die mittlere Teleskophülse 11 eingeschoben. Dies geschieht solange, bis die Rasthaken 52 der mittleren Teleskophülse 11 in Ausnehmungen 61 der Verbindungs-Teleskophülse 12 zu liegen kommen, die wiederum an Ende von axialen Führungsbahnen 60 in einer Außenwand der Verbindungs-Teleskophülse 12 ausgeführt sind.

Sodann wird der Formschluss-Adapter 13, wiederum in Richtung des Pfeils 32, in die Verbindungs-Teleskophülse 12 eingeschoben, bis die Rasthaken 46 der Verbindungs-Teleskophülse 12 von außen in Ausnehmungen 62 des Formschluss-Adapters 13 eingreifen. Die Ausnehmungen 62 sind wiederum in axialen Führungsbahnen 60 des Formschluss-Adapters 13 ausgeführt. In dieser vormontierten Position sind die Hülsen 11 und 12 praktisch vollständig in der äußeren Schutz-Teleskophülse 10 angeordnet. Der Formschluss-Adapter 13 steht mit dem größten Teil einer Axialerstreckung zwischen den Ausnehmungen 62 und dem Anlagebund 45 über die ineinandergeschobenen Teleskophülsen 10 bis 12 über.

Bei der Montage der vormontierten Stechschutzeinrichtung 8 mit der Injektionseinrichtung 2 wird die Stechschutzeinrichtung 8 mit führendem Formschluss-Adapter 13 auf den Mündungsabschnitt 6 der Injektionseinrichtung 2 aufgeschoben, bis der Anlagebund 45 am Rastbund 25 des Mündungsabschnitts 6 anschlägt. Anschließend werden die drei ineinandergeschobenen Teleskophülsen 10 bis 12 in axialer Richtung weiter auf den Behälter 3 zu verlagert, wobei die Rasthaken 46 der Verbindungs-Teleskophülse 12 aus den Ausnehmungen 62 des Formschluss-Adapters 13 ausrücken und zunächst längs der Führungsbahnen 60 gleiten und sodann den Rastbund 25 zur Sicherung der Stechschutzeinrichtung 8 an der Injektionseinrichtung 2 hintergreifen. Gleichzeitig sorgen wiederum Niederhaltemittel dafür, dass die Verdrehschutz-Zungen 47 des Formschluss-Adapters 13 zwischen benachbarten Umfangsrippen 29 des Mündungsabschnitts 6 zum Verdrehschutz der Stechschutzeinrichtung 8 an der Injektionseinrichtung 2 niedergehalten werden.

Das Zusammenwirken der Führungsbahnen 60 mit den zugeordneten Rasthaken 46, 52 dient als Verdrehschutz der Komponenten der Stechschutzeinrichtung 8 relativ zueinander. Eine weitere Verdrehsicherung ist durch Axialführungen jeweils um 90° versetzt zu den Rasthaken/Führungsbahnen-Gestaltungen gegeben.

Die Stechschutzeinrichtung 8 ist dann vorbereitet in der Injektionsstellung.

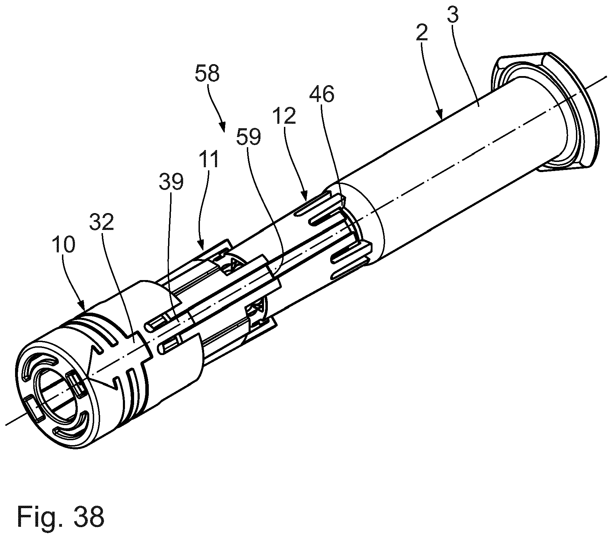

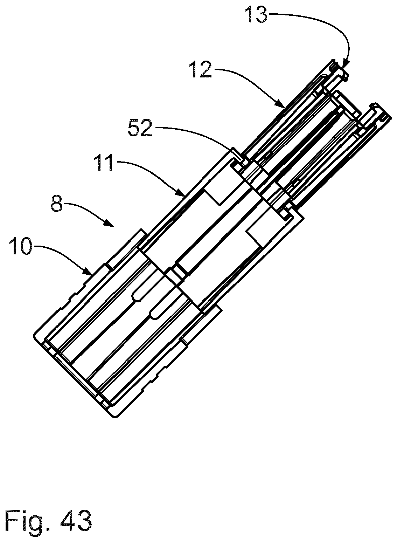

Beim Überführen der Stechschutzeinrichtung 8 von der Injektionsstellung in die Schutzstellung rücken einerseits die Rasthaken 52 der mittleren Teleskophülse 11 aus den Ausnehmungen 61 der Verbindungs-Teleskophülse 12 und andererseits die Rasthaken 52 der äußeren Schutz-Teleskophülse 10 aus den Gegenausnehmungen 59 der mittleren Teleskophülse 11 aus. Die Rasthaken der Teleskophülsen 10, 11 laufen dann axial längs den jeweiligen Führungsbahnen 60 der Teleskophülsen 11 und 12, bis die Rasthaken 52 der äußeren Schutz-Teleskophülse 10 in Ausnehmungen 63 einrücken, die an den Gegenausnehmungen 59 gegenüberliegenden Enden der Führungsbahnen 60 ausgeführt sind. In der Schutzstellung rücken weiterhin die Rasthaken 52 der mittleren Teleskophülse 11 in Ausnehmungen 63 ein, die an den Ausnehmungen 61 gegenüberliegenden Enden in den Führungsbahnen 60 der Verbindungs-Teleskophülse 12 ausgeführt sind. Somit ist die teleskopierte Schutzstellung nach Fig. 38 oder 43 erreicht.

## Patentansprüche

1. Medizinische Injektionsvorrichtung (1; 33; 41; 56; 58)
- mit einer Injektionseinrichtung (2), umfassend:
-- einen Behälter (3) für das zu injizierende Medium,
-- eine Injektionskanüle (4), die mit dem Behälter (3) kommuniziert,
- mit einer Stechschutzeinrichtung (8), die verlagerbar ist zwischen
-- einer Injektionsstellung, in der die Injektionskanüle (4) zum Injizieren des Mediums freilegbar ist,
-- einer Schutzstellung, in der eine Kanülenspitze (9) der Injektionskanüle (4) in einer Schutzkomponente (10; 34) der Stechschutzeinrichtung (8) versenkt angeordnet ist,
- mit einer Sicherungseinrichtung (14; 39, 40) zum sichernden Festlegen der Schutzkomponente (10; 34) in der Schutzstellung,
- mit einem Formschluss-Adapter (13; 36) zur Formschlussverbindung der Stechschutzeinrichtung (8) mit der Injektionseinrichtung (2),
- wobei die Stechschutzeinrichtung (8) mindestens zwei Teleskophülsen (10 bis 12; 34 bis 36; 34, 35, 42) aufweist, wobei eine Verbindungs-Teleskophülse (12; 36; 42) mit der Injektionseinrichtung (2) verbunden ist und wobei eine Schutz-Teleskophülse (10; 34) die Schutzkomponente darstellt,
**dadurch gekennzeichnet, dass**
- mindestens ein Bauteil der Stechschutzeinrichtung (8) als Mehrkomponenten-Spritzgussteil ausgeführt ist und
- mindestens ein Mehrkomponenten-Spritzguss-Bauteil der Stechschutzeinrichtung (8) als äußere Hülse (10) ausgeführt ist, die mindestens einen Griffabschnitt (55) und mindestens einen Tragkörper (54) aufweist, wobei der Tragkörper (54) einerseits und der mindestens eine Griffabschnitt (55) andererseits als unterschiedliche Spritzguss-Komponenten des Mehrkomponenten-Spritzguss-Bauteils ausgeführt sind.

2. Injektionsvorrichtung nach Anspruch 1, **dadurch gekennzeichnet, dass** die Sicherungseinrichtung (14; 39, 40) als Schutz-Rastanordnung zum rastenden Festlegen der Schutzkomponente (10; 34) in der Schutzstellung ausgeführt ist.

3. Injektionsvorrichtung nach Anspruch 1 oder 2, **gekennzeichnet durch** eine Injektions-Verbindungsanordnung (18, 24; 46) zum formschlüssigen Festlegen der Schutzkomponente (10; 34) an der Injektionseinrichtung (2) in der Injektionsstellung.

4. Injektionsvorrichtung nach Anspruch 3, **dadurch gekennzeichnet, dass** die Injektions-Verbindungsanordnung (18, 24) als Injektions-Rastanordnung zum rastenden Festlegen der Schutzkomponente (10; 34; 46) in der Injektionsstellung ausgelegt ist.

5. Injektionsvorrichtung nach einem der Ansprüche 1 bis 4, **dadurch gekennzeichnet, dass** zwischen der Verbindungs-Teleskophülse (12; 36; 42) und der Schutz-Teleskophülse (10; 34) mindestens eine weitere Teleskophülse (11; 35) der Stechschutzeinrichtung (8) angeordnet ist.

6. Injektionsvorrichtung nach einem der Ansprüche 1 bis 5, **dadurch gekennzeichnet, dass** die Schutz-Rastanordnung (14) mindestens eine Rast-Zahnreihe (15) hintereinander längs mindestens einer der Teleskop-Hülsen (11, 12) angeordneter Rastzähne (16; 16') aufweist, in die ein Gegenrastkörper (17, 18) der dieser Teleskop-Hülse (11, 12) benachbarten weiteren Teleskop-Hülse (10, 11) der Stechschutzeinrichtung (8) eingreift.

7. Injektionsvorrichtung nach einem der Ansprüche 1 bis 6, **gekennzeichnet durch** mindestens eine Nut/Feder-Führungseinrichtung (19) zur Gewährleistung einer Teleskop-Führung und eines Verdrehschutzes zwischen zwei benachbarten Teleskophülsen (10 bis 12; 34 bis 36; 34, 35, 42).

8. Injektionsvorrichtung nach einem der Ansprüche 1 bis 7, **dadurch gekennzeichnet, dass** die Stechschutzeinrichtung (8) aufweist:
- mindestens ein Formschluss-Element (37), das zwischen zwei benachbarten, axial verlaufenden Umfangsrippen (29) eines Mündungs- und Verbindungsabschnitts (6) der Injektionseinrichtung (2) aufgenommen ist,
- wobei das Formschluss-Element (37) an einem Formschluss-Element-Tragkörper (36) angeformt ist,
- wobei der Formschluss-Element-Tragkörper (36) einerseits und das mindestens eine Formschluss-Element (37) andererseits als unterschiedliche Spritzguss-Komponenten des Mehrkomponenten-Spritzguss-Bauteils (36) ausgeführt sind.

## Claims

1. Medical injection device (1; 33; 41; 56; 58), comprising:
- an injection unit (2), comprising:
-- a container (3) for the medium to be injected,
-- an injection cannula (4), which communicates with the container (3),
- a needle guard (8), which is displaceable between
-- an injection position, in which the injection cannula (4) is adapted to be opened up to inject the medium, and
-- a safe position, in which a cannula tip (9) of the injection cannula (4) is arranged recessed in a protective component (10; 34) of the needle guard (8),
- a securing device (14; 39, 40) for securely fixing the protective component (10; 34) in the safe position,
- an interlocking adapter (13; 36) for connecting the needle guard (8) to the injection unit (2) in an interlocking connection,
- wherein the needle guard (8) has at least two telescoping sleeves (10 to 12; 34 to 36; 34, 35, 42), wherein a connecting telescoping sleeve (12; 36; 42) is connected to the injection unit (2) and wherein a protective telescoping sleeve (10; 34) forms the protective component, **characterized in that**
- at least one part of the needle guard (8) is designed as a multi-component injection-molded part, and
- at least one multi-component injection-molded part of the needle guard (8) is designed as an outer sleeve (10), which has at least one grip section (55) and at least one supporting body (54), wherein the supporting body (54) on the one hand and the at least one grip section (55) on the other hand are designed as different injection-molded components of the multi-component injection-molded part.

2. Injection device according to claim 1, **characterized in that** the securing device (14; 39, 40) is designed as a guard locking assembly for locking the protective component (10; 34) in the safe position.

3. Injection device according to claim 1 or 2, **characterized by** an injection connecting assembly (18, 24; 46) for fixing the protective component (10; 34) on the injection unit (2) in the injection position in an interlocking manner.

4. Injection device according to claim 3, **characterized in that** the injection connecting assembly (18, 24) is designed as an injection locking assembly for locking the protective component (10; 34; 46) in the injection position.

5. Injection device according to any one of claims 1 to 4, **characterized in that** at least one additional telescoping sleeve (11; 35) of the needle guard (8) is arranged between the connecting telescoping sleeve (12; 36; 42) and the protective telescoping sleeve (10; 34).

6. Injection device according to any one of claims 1 to 5, **characterized in that** the guard locking assembly (14) has at least one locking teeth row (15) of locking teeth (16; 16) arranged one in front of the other along at least one of the telescoping sleeves (11, 12), with a counter locking body (17, 18) of the additional telescoping sleeve (10, 11), which is adjacent to said telescoping sleeve (11, 12), of the needle guard (8) engaging into said locking teeth row (15).

7. Injection device according to any one of claims 1 to 6, **characterized by** at least one tongue/groove guide device (19) for ensuring a telescoping guideway and rotation prevention between two adjacent telescoping sleeves (10 to 12; 34 to 36; 34, 35, 42).

8. Injection device according to any one of claims 1 to 7, **characterized in that** the needle guard (8) has:
- at least one interlocking element (37), which is held between two adjacent, axially extending peripheral ribs (29) of an opening and connecting section (6) of the injection unit (2),
- wherein the interlocking element (37) is formed on an interlocking element supporting body (36),
- wherein the interlocking element supporting body (36) on the one hand and the at least one interlocking element (37) on the other hand are designed as different injection-molded components of the multi-component injection-molded part (36).

## Revendications

1. Dispositif d'injection à usage médical (1 ; 33 ; 41 ; 56 ; 58) comportant
- un moyen d'injection (2), comprenant :
-- un récipient (3) destiné au milieu à injecter,
-- une canule d'injection (4) communiquant avec le récipient (3),
- un moyen anti-piqûre (8) déplaçable entre
-- une position d'injection dans laquelle la canule d'injection (4) peut être libérée pour injecter le milieu,
-- une position de protection dans laquelle une pointe (9) de la canule d'injection (4) est disposée de manière escamotée dans un composant de protection (10 ; 34) du moyen anti-piqûre (8),
- un moyen de sécurité (14 ; 39, 40) destiné à immobiliser de manière sûre le composant de protection (10 ; 34) dans la position de protection ;
- un adaptateur à complémentarité de formes (13 ; 36) destiné à raccorder par complémentarité de formes le moyen anti-piqûre (8) au moyen d'injection (2),
- le moyen anti-piqûre (8) comprenant au moins deux manchons télescopiques (10 à 12 ; 34 à 36 ; 34, 35, 42), un manchon télescopique de raccordement (12 ; 36 ; 42) étant raccordé au moyen d'injection (2), et un manchon télescopique de protection (10 ; 34) représentant le composant de protection,
**caractérisé en ce que**
- au moins un composant du moyen anti-piqûre (8) se présente sous la forme d'une pièce moulée par injection à plusieurs composants et
- au moins un élément structurel moulé par injection à plusieurs composants du moyen anti-piqûre (8) se présente sous la forme d'un manchon extérieur (10) qui comporte au moins une partie de préhension (55) et au moins un corps de support (54), le corps de support (54) d'une part et l'au moins une partie de préhension (55) d'autre part se présentant sous la forme de différents composants moulés par injection de l'élément structurel moulé par injection à plusieurs composants.

2. Dispositif d'injection selon la revendication 1, **caractérisé en ce que** le moyen de sécurité (14 ; 39, 40) se présente sous la forme d'un ensemble d'encliquetage de protection destiné à immobiliser par encliquetage le composant de protection (10 ; 34) dans la position de protection.

3. Dispositif d'injection selon la revendication 1 ou 2, **caractérisé par** un ensemble de liaison et d'injection (18, 24 ; 46) destiné à immobiliser par complémentarité de formes le composant de protection (10 ; 34) au niveau du moyen d'injection (2) dans la position d'injection.

4. Dispositif d'injection selon la revendication 3, **caractérisé en ce que** l'ensemble de liaison et d'injection (18, 24) est conçu comme un ensemble d'encliquetage et d'injection destiné à immobiliser par encliquetage le composant de protection (10 ; 34 ; 46) dans la position d'injection.

5. Dispositif d'injection selon l'une des revendications 1 à 4, **caractérisé en ce qu'**au moins un autre manchon télescopique (11 ; 35) du moyen anti-piqûre (8) est disposé entre le manchon télescopique de raccordement (12 ; 36 ; 42) et le manchon télescopique de protection (10 ; 34).

6. Dispositif d'injection selon l'une des revendications 1 à 5, **caractérisé en ce que** l'ensemble d'encliquetage et de protection (14) comporte au moins une rangée (15) de dents d'encliquetage (16 ; 16') qui sont disposées les unes derrière les autres le long d'au moins un des manchons télescopiques (11, 12), dans lesquelles s'engrène un corps d'encliquetage homologue (17, 18) de l'autre manchon télescopique (10, 11), adjacent à ce manchon télescopique (11, 12), du moyen anti-piqûre (8).

7. Dispositif d'injection selon l'une des revendications 1 à 6, **caractérisé par** au moins un moyen de guidage à ressort et rainure (19) destiné à assurer un guidage télescopique et une protection anti-rotation entre deux manchons télescopiques adjacents (10 à 12 ; 34 à 36 ; 34, 35,42).

8. Dispositif d'injection selon l'une des revendications 1 à 7, **caractérisé en ce que** le moyen anti-piqûre (8) comporte :
- au moins un élément de liaison par complémentarité de formes (37) reçu entre deux nervures circonférentielles (29) adjacentes s'étendant axialement d'une partie de raccordement et d'embouchure (6) du dispositif d'injection (2),
- l'élément de liaison par complémentarité de formes (37) étant formé au niveau d'un corps de support d'élément de liaison par complémentarité de formes (36),
- le corps de support d'élément de liaison par complémentarité de formes (36) d'une part et l'au moins un élément de liaison par complémentarité de formes (37) d'autre part se présentant sous la forme de différents composants moulés par injection de l'élément structurel (36) moulé par injection à plusieurs composants.
